(19) Europäisches Patentamt
European Patent Office
Office européen des brevets

(11) Veröffentlichungsnummer: **0 219 616**
**A1**

(12) # EUROPÄISCHE PATENTANMELDUNG

(21) Anmeldenummer: **86110018.8**

(51) Int. Cl.[4]: **C07D 217/20** , //A61K31/485

(22) Anmeldetag: **21.07.86**

Die Bezeichnung der Erfindung wurde geändert (Richtlinien für die Prüfung im EPA, A-III, 7.3).

(30) Priorität: **19.07.85 BG 71158/85**

(43) Veröffentlichungstag der Anmeldung:
**29.04.87 Patentblatt 87/18**

(84) Benannte Vertragsstaaten:
**AT BE CH DE FR GB IT LI NL SE**

(71) Anmelder: **S O "PHARMACHIM"**
**Ilensko Chaussée 16**
**Sofia(BG)**

(72) Erfinder: **Ivanov, Tschavdar Borissov**
**Komplex Mladost, Bl. 46-4**
**Sofia(GB)**
Erfinder: **Mondeschka, Diana Minkova**
**Rakovski-Strasse 123**
**Sofia(BG)**
Erfinder: **Stoev, Georgi Georgiev**
**Komplex Mladost-I, Bl. 44-1**
**Sofia(BG)**
Erfinder: **Mizov, Vladimir Sdravkov**
**Komplex Mladost-II, Bl. 215-5**
**Sofia(BG)**
Erfinder: **Sabunova,, Orhideya Bontscheva**
**Gerlovo-Strasse 15**
**Sofia(BG)**
Erfinder: **Batschvarova, Anna Siderova**
**6. Septemvri-Strasse 40**
**Sofia(BG)**
Erfinder: **Todorova, Maria Georgieva**
**Komplex Lenin, Bl. 32-1**
**Sofia(BG)**
Erfinder: **Genova, Ani Iavnova**
**Postoyanstvo-Strasse 38**
**Sofia(BG)**
Erfinder: **Spirova, Neli Stefanova**
**Han Krum-Strasse 4-5**
**Sofia(BG)**
Erfinder: **Stoimenov, Georgi Borissov**
**K. Vesskova-Strasse, Bl. 238**
**Sofia(BG)**
Erfinder: **Iliev, Dimiter Pavlov**
**Komplex Mladost, Bl. 96-1**
**Sofia(BG)**
Erfinder: **Nissimov, Yossif Nissim**
**Sofronii-Strasse 37**
**Sofia(BG)**
Erfinder: **Taneva, Nedyalka Ivanova**
**Quartal E. Markov, Bl. 249-3**
**Sofia(BG)**
Erfinder: **Spirov, Georgi Georgiev**
**Han Krum-Strasse 4-5**
**Sofia(BG)**

EP 0 219 616 A1

Erfinder: **Vassileva, Raina Stefanova**
**Emine-Strasse 10-2**
**Sofia(BG)**

(74) Vertreter: **von Füner, Alexander, Dr. et al**
**Patentanwälte v. Füner, Ebbinghaus, Finck**
**Mariahilfplatz 2 & 3**
**D-8000 München 90(DE)**

(54) **N,N'-Dimethyl-N,N'-4,10-dioxa-3,11-dioxo-1,13-tridecylen-bis-tetrahydropapaveriniumdichlorid.**

(57) Dichlormethylat des N,N-4,10-Dioxa-3,11-dioxotridezylen-1, 13-bis-tetrahydropapaverin der allgemeinen Formel I

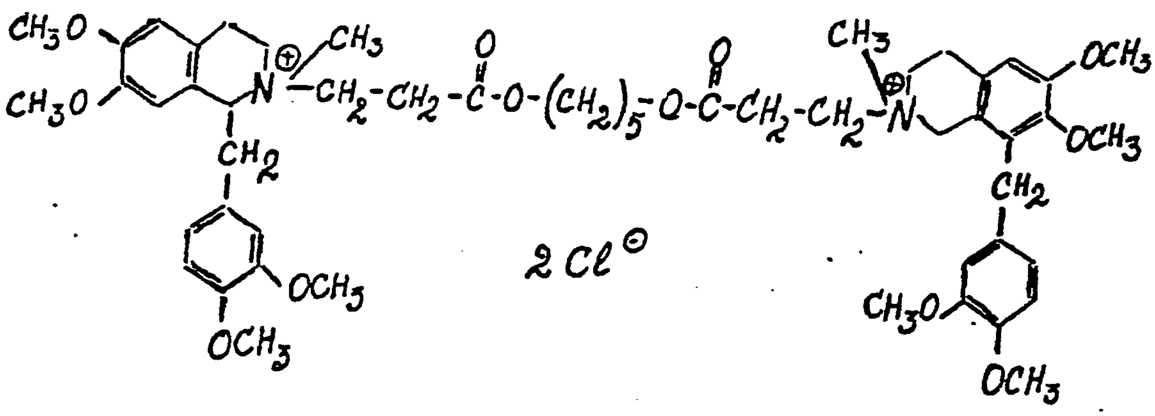

sowie ein Verfahren zu seiner Herstellung.

# DICHLORMETHYLAT DES N,N-4,10-DIOXA-3,11-DIOXOTRIDEZYLEN-4,10-BIS-TETRAHYDROPAPAVERINS UND EIN VERFAHREN ZU SEINER HERSTELLUNG

Die Erfindung betrifft ein Dichlormethylat des N,N-4,10-Dioxa-3,11-dioxotridezylen-1,13-bis-tetrahydropapaverins und ein Verfahren zu seiner Herstellung.

Es ist bekannt, daß die quaternären Salze der Bis-Benzylisochinolin polyalkylen-diester der allgemeinen Formel

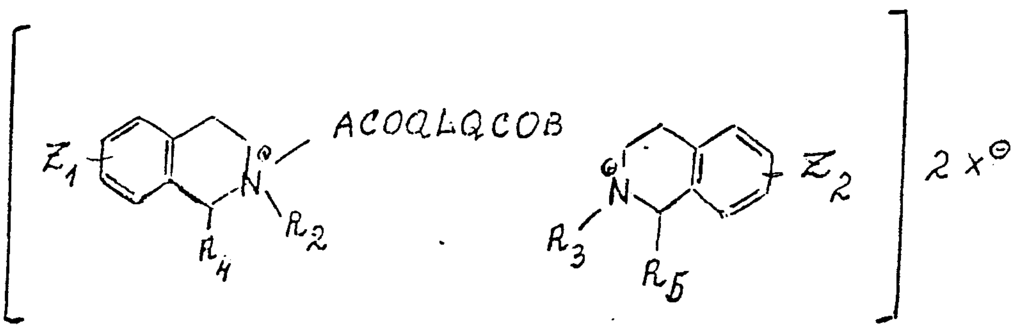

ausgeprägte neuromuskel-blockierende Eigenschaften aufweisen (1,2), wo $Z_1$ und $Z_2$ die Methylendioxy-Gruppe oder eine bis drei Methoxy-Gruppen bedeuten,

$R_2$ und $R_3$ Alkylgruppen mit ein bis drei Kohlenstoffatomen bedeuten, $R_4$ und $R_5$ eine Benzyl-oder Phenylgruppe bedeuten, wobei die Phenylgruppe durch Halogen, Alkoxy-oder Methylendioxy-Gruppen substituiert ist

A und B Alkylenreste, enthaltend 1, 2 oder 3 Kohlenstoffatome, bedeuten,

L eine Alkylenkette bedeutet, die von 2 bis 12 Kohlenstoffatome enthält oder eine Gruppe $L^1$, $L^2$ in der jede $L^1$ und $L^2$ alkylenenthaltend mit zwei Kohlenstoffatomen ist, und die Summe von $L^1$ und $L^2$ bis 11 Kohlenstoffatome aufweist und

X anorganisches oder organisches Anion bedeutet.

Von diesen Salzen hat das Dibesylat des N,N-4,10-Dioxa-3,11-Dioxotridezylen-1,13-bis-tetrahydropapaverins als Präparat unter der Handelsbezeichnung "Tracrium" Anwendung gefunden, welches den Nachteil aufweist, daß es mit 8% Verunreinigungen hergestellt wird. Dies erfordert, daß bei der Herstellung der Arzneiform größere Mengen des Präparats verwendet werden, wodurch die Toxizität des Präparats potentiellerhöht wird und es eine geringe Haltbarkeit besitzt. Das Dichlormethylat des N,N-4,10-Dioxa-3,11-dioxotridezylen-1,13-bis-tetrahydropapaverins ist bis jetzt noch nicht hergestellt und beschrieben worden, da dieses Salz nach dem unter (1) beschriebenen Verfahren praktisch nicht herzustellen ist.

Aufgabe der Erfindung war es nun, ein neues Salz des N-N-4,10-Dioxa-3,11-dioxotridezylen-1,13-bis-tetrahydropapaverins zu synthesieren und ein Verfahren zu seiner Herstellung zu erarbeiten, das eine höhere Aktivität, erhöhte Stabilität und niedrigeren Gehalt an ver unreinigenden Nebenprodukten aufweist.

Diese Aufgabe wurde erfindungsgemäß durch ein Dichlormethylat des N,N-4,10-Dioxa-3,11-dioxotridezylen-1,13-bis-tetrahydropapaverins gelöst. Das neue Salz weist erfindungsgemäß folgende allgemeine Formel auf:

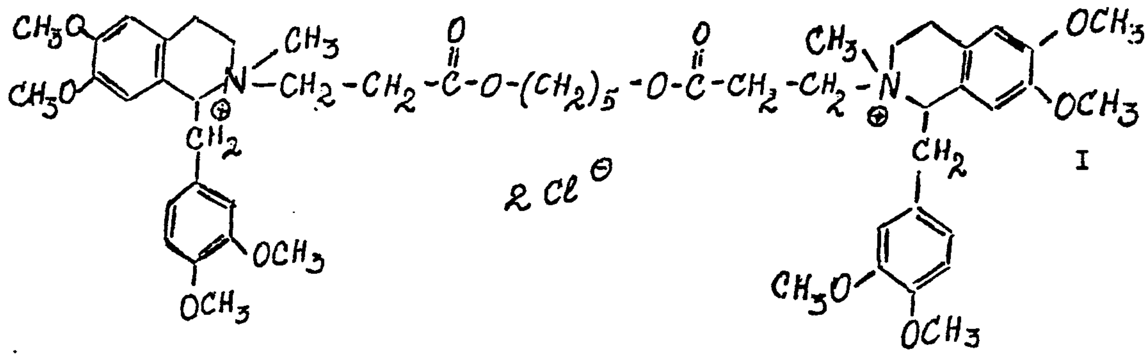

I

Das Verfahren zur Herstellung der Verbindung der Formel I besteht darin, daß N-/2-Carboxyäthyl/-1-/3,4-dimethoxybenzyl/-6,7-dimethoxy-1,2,3,4-tetrahydroisochinolin der Formel II

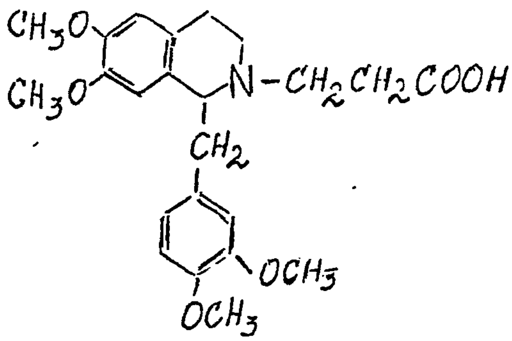

II

mit 1,5-Pentamethylendiol in Anwesenheit von sauren Katalysatoren in einem Medium aromatischer Kohlenwasserstoffe unter azeotroper Ausscheidung des Reaktionswassers bis zur Herstellung des Diesters IV verestert, welcher auf bekannte Weise mit Methyljodid zum N,N-Dimethyl-N,N-4,10-dioxa-3,11-dioxotridezylen-1,13-bis-tetrahydropapaverinoildijodid V quaternärisiert wird und durch Anionenaustausch unter Verwendung starkbasischer Anionenaustauscher-Harze in eine Verbindung der Formel I umgesetzt wird. Die sauren Katalysatoren können organische oder anorganische Säuren, sein, vorzugsweise p-Toluolsulfonsäure.

Die Veresterung des 1,5-Pentamethylendiols wird mit einem Überschuß der Verbindung der Formel II in Molverhältnissen Säure zu Diol von 2:1 bis 4:1 durchgeführt. Das Molverhältnis der p-Toluolsulfonsäure zu Verbindung der Formel II beträgt von 1:1 bis 2:1. Die Herstellung des Oxalatsalzes des Diesters wird in einem Azetonmedium durchgeführt. Die starkbasischen Anionenaustauscher-Harze können Dowex®, Amberlits® oder Amberlists® in Chloridform sein, und als Eluente bei dem Anionenaustausch aliphatische Alkohole, aliphatische Ketone oder aliphatische Nitrile benutzt werden. Der Austausch kann in einem stationären Zustand oder in einer chromatografischen Säule durchgeführt werden.

Das Verfahren verläuft nach folgendem Schema :

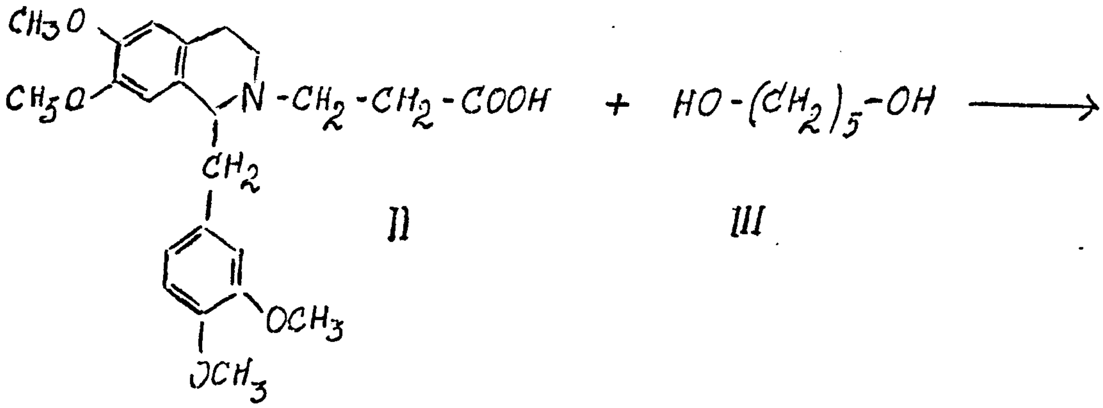

II III

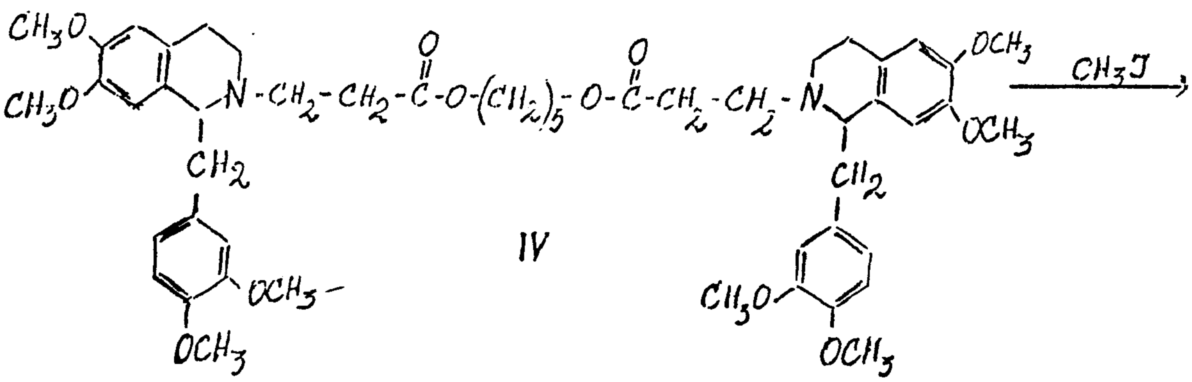


IV

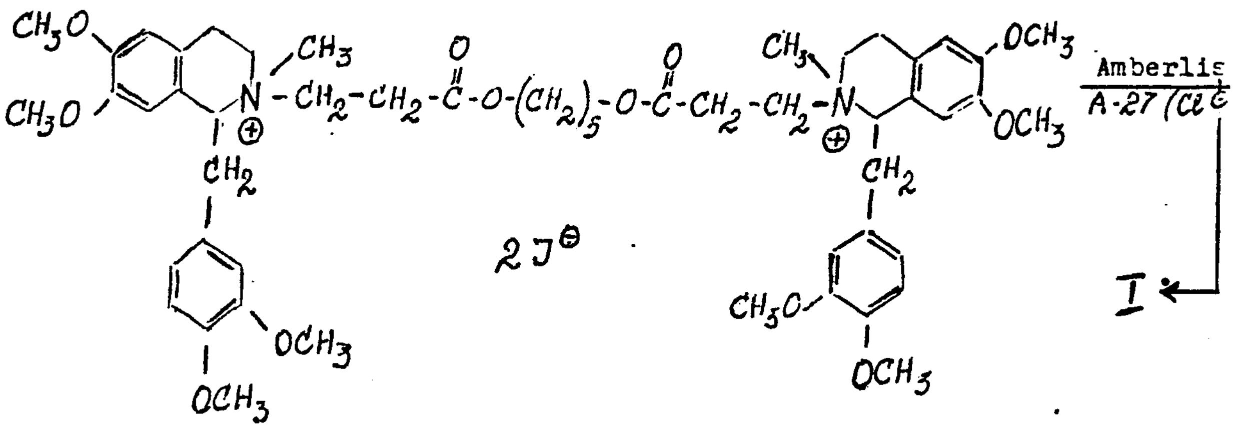


I

V

Die Veresterung des Diols III wird bei einem Überschuß der Säure II bei einem Molverhältnis Säure -Diol von 2:1 bis 4:1 durchgeführt. Als saure Katalysatoren können Lewis-Säuren, organische oder anorganische Säuren, vorzugsweise p-Toluolsulfonsäure,in Überschuß verwendet werden, wobei das Verhältnis p-Toluolsulfonsäure zur Verbindung der Formel II von 1:1 bis 2:1 beträgt.

Als Reaktionsmedium können aromatische Kohlenstoffe (Benzol, Toluol, Xylol) verwendet werden.

Die Veresterung wird bei Siedetemperatur des Reaktionsgemisches mit azeotroper Wasserausscheidung während einer bis 20 Stunden durchgeführt. Nach Beenden der Veresterung wird das in Ölform hergestellte Produkt in einem Gemisch aus Ethanol und Wasser (2:1) gelöst, wonach die hergestellte Lösung mit einer 10%-igen Natriumbikarbonat-Lösung bis zu einem pH-Wert 7,5 alkalisiert wird. Das ausgeschiedene Öl wird mit Chloroform extrahiert, dann mit Wasser gespült und das organische Extrakt bis zu Trockenmasse

verdampft. Der hergestellte rohe Diester (freie Base) der Formel IV wird in Oxalat mittels Auflösen in Azeton und Beifügen in der Wärme einer Azetonlösung von Oxalsäure umgesetzt. Das so hergestellte Oxalat der Verbindung mit der Formel IV wird zweimal aus einem Gemisch von Wasser und Azeton umkristallisiert.

Das Ausgangs-N-/2-Carboxyäthyl/-1-/3,4-dimethoxybenzyl/-6,7-dimethoxy-1,2,3,4-tetrahydroisochinolin der Formel II stellt eine neue Verbindung dar, die nach folgendem Verfahren hergestellt wird : Tetrahydropapaverin und Beta-Propiolakton werden in einem Azetonmedium unter Sieden des Reaktionsgemisches während 3 Stunden erhitzt und das hergestellte rohe Produkt wird durch Umkristallisieren aus Azeton gereinigt.

Das Dijodid ist nach dem unter (1,2) beschriebenen Verfahren durch Qua ternärisieren des Diesters IV (freie Base) mit einem Überschuß von Methyljodid in einem Medium aus trockenem Azetonnitril bei Zimmertemperatur während 22 Stunden hergestellt worden.

Die Herstellung der Verbindung der Formel I erfolgt durch einen Anionenaustausch des Dijodids V unter Verwendung von starkbasischen Anionenaustauscher-Harzen vom Dowextyp , Amberlits oder Amberlists in Chloridform, vorzugsweise Amberlist A-26® und Amberlist A-27®. Der Ionenaustauschprozeß kann sowohl im stationären Zustand unter Umrühren, als auch in einer chromatografischen Säule durchgeführt werden. Als Eluente können aliphatische Alkohole, Ketone oder Nitrile benutzt werden. Der Ionenaustausch kann sowohl mit isoliertem Dijodid, als auch ohne seine Isolierung durchgeführt werden, wobei im letzten Fall die nach der Beendigung des Quaternärisierens erhaltene Lösung des Dijodids bis zu einem bestimmten Volumen zur Beseitigung des überschüssigen Methyljodids konzentriert wird, wonach das Konzentrat mit frischem Lösungsmittel verdünnt und durch die Säule durchgelassen wird.

Das Isolieren des Dichlormethylats der Formel I erfolgt durch Fällung aus trockenem Äther oder aus hochsiedenden Paraffin-Kohlenwasserstoffen.

Die Verbindung der Formel I weist vier asymetrische Zentren auf, je zwei für jeden Tetrahydroisochinolin-Ring, weswegen sie ein Gemisch aus Stereoisomeren darstellt, analog dem bekannten Dibezylat.

Die Struktur von I ist durch ihre $^1$H-Kernmagnetische Resonanz-Spektren und Elementeranalyse festgestellt, und ihr Prozentgehalt und Vorhandensein von Verunreinigungen -durch hocheffektive Flüssig-Chromatografie.

Es wurde unerwartet festgestellt, daß das neue Salz wesentlich bessere Eigenschaften für die Anwendung als Arzneimittel aufweist im Vergleich mit den bekannten Salzen derselben Gruppe: Die Verbindung der Formel I enthält wesentlich weniger Nebenprodukte (1 bis 2%) im Vergleich zum Bezylat (bis 9%) , bzw. der Prozentgehalt ist höher und erreicht 98-99% nach den aus der hocheffektiven Flüssig-Chromatografie erhaltenen Daten. Im Vergleich zum Bezylat, bei dessen Synthese sechs begleitende Nebenprodukte gebildet werden, von welchen vier Abbauprodukte und zwei Strukturanaloge sind mit einer zulässigen Totalmenge von 8 bis 9% (3). Im Dichlorid sind nur bis zu 2% Chlormethylat des N-/2-Carboxyäthyl/-1,2,3,4-tetrahydropapaverins und Laudanozin-Spuren enthalten.

Die Verbindungen der Formel I besitzen eine bessere Löslichkeit im Vergleich zu dem bekannten Dijodid und den anderen bis jetzt hergestellten und untersuchten Salzen derselben Gruppe, was von Vorteil für die Herstellung von Ampullen ist.Sie weist eine erhöhte relative biologische Aktivität auf. Dank ihrer hohen chemischen Reinheit im Vergleich zu dem bis jetzt in der Arzneipraxis benutzten Bezylat, da es nicht erforderlich ist zusätzliche Mengen vom Aktivstoff in die Ampulle beizufügen und zwar 10 mg/ml im Vergleich zu 10,9 mg/ml vom Bezylat.

Bei einer Vergleichsprüfung der Stabilität der Verbindung der Formel I und des Bezylats wurde festgestellt, daß das Dichlormethylat wesentlich stabiler ist. Die Stabilität wurde mittels der hochaktiven Flüssig-Chromatografie in wässrig-sauren Medium in einem Bereich der pH-Werte von 1,7 bis 4,0 (Tabelle 1) geprüft. Die Prüfung wurde bei unterschiedlichen pH-Werten in dem angegebenen Intervall durch Behandlung der wässrigen Lösungen des Dichlormethylats der Formel I und des Bezylats bei einer Temperatur von 53°C während 40 Stunden durchgeführt. Die erhaltenen Ergebnisse zeigen, daß der Prozentgehalt von I in einem Interval der pH-Werte von 2,46 bis 3,77 von 86 bis 91,9% mit Maximum 91,9% bei pH-Werten 3,0 -3,27 beträgt. Demgegenüber ist der Prozentgehalt des diesseits auf bekannte Weise synthetisierten Bezylats bei demselben Bereich der pH-Werte 72 bis 74% mit Maximum 73,79 bei einem pH-Wert von 3,50, d.h. mit zirka 20% niedriger im Vergleich zur Verbindung der Formel I , was für eine wesentliche höhere Stabilität der Verbindung mit Formel I gegenüber der sauren Hydrolyse spricht.Ähnliche Ergebnisse über die Stabilität des Bezylats sind unter (4) beschrieben, bei welchem die maximale Stabilität bei einem pH-Wert von 2,5 (Prozentgehalt des Bezylats 76%) erhalten wurde.

Die Verbindung der Formel I zeigte bei den durchgeführten pharmakologischen Prüfungen einen verbesserten biologischen Effekt, da mit einem kleineren Quantum des Aktivstoffes derselbe Effekt erzielt wird. Anhand der nachstehend angeführten

Beispiele wird die Erfindung näher erläutert:

Beispiel 1. Herstellung von N-/2-carboxyäthyl/-1-/3,4-dimethoxybenzyl/- 6,7-dimethoxy-1,2,3,4-tetrahydroisochinolin.

Zu 39 g (0,113 Mol) in 390 ml Azeton gelösten Tetrahydropapaverin (freie Base) werden tropfenweise für cirka 30 Minuten 9,66 g (0,134 Mol) Beta-Propiolakton in 390 ml Azeton zugegeben. Das Gemisch wird während 3 Stunden erhitzt. Danach wird es abgekühlt und zum Kristallisieren während 24 Stunden stehen gelassen. Der erhaltene weiße kristalline Niederschlag wird abfiltriert und dann mit Azeton gespült. Nach dem Trocknen bei 60°C werden 36,4 g (76%) des N-Carbooxyäthyl-Derivats erhalten, das aus Azeton umkristallisiert wird.

Ausbeute: 23,6 g mit einem Schmelzpunkt von 125,5 bis 126,5°C.

Elementenanalyse -berechnet in % C 66,49, N 3,73, H 7,03

gefunden in % C 66,53, N 3,19, H 7,34

$^1$H kernmagnetische Resonanz ($CDCl_3$) δ; ppm : 2,32-3,80

(m,10H), N $CH_2CH_2CO$,3-$CH_2$,4-$CH_2$ und $ArCH_2$, 3,47 (s,3H), 3,72 (s,3H), 3,76 (s,6H), 4x$CH_3$0,5,79 - (s,1H), 8-CH, 6,45 (s,1H), 5-CH,6,42-6,76 (m,3H), 3ArH, 9,80 (erweitertes Singulett, wird mit $D_2O$ ausgetauscht), COOH.

Beispiel 2. Herstellung von N,N-4,10 Dioxa-3,11-dioxotridezylen-1,13-bis-tetrachydropapaverin Dioxalat. Zu 400 ml trockenem Toluol werden 22 g (0,052 Mol) N-/2-carboxyäthyl/-1-(3,4-dimethoxybenzyl)-6,7-dimethoxy-1,2,3,4-tetrahydroisochinolin, 2,04 g (0,02 Mol) 1,5-Pentamethylendiol, 14 g (0,074 Mol) p-Toluolsulfonsäure-monohydrat beigefügt, wonach das hergestellte Gemisch bis zum Sieden während 10 Stunden bei azeotroper Ausscheidung des Reaktionswassers erhitzt wird. Nach Ablauf der Reaktionszeit wird das Gemisch bis zur Zimmertemperatur abgekühlt. Die Toluolschicht wird von dem sich bildenden ölartigen Produkt dekantiert, welches in 600 ml Ethanol-Wasser-Gemisch (2:1) teilweise gelöst wird. Es werden 100 ml Toluol zugegeben und mit einer 10%-igen Natriumbicarbonat-Lösung bis zu einem pH-Wert von zirka 7,5 alkalisiert. Die Toluolschicht wird abgeschieden, während die wässrig-alkoholische Schicht noch drei Mal mit je 100 ml Toluol extrahiert wird. Die Toluolextrakte werden mit 3x200 Wasser gespült, auf Natriumsulfat getrocknet, wonach das Lösungsmittel bis zum Trocknen abdestiliert wird. Der erhaltene hellgelbe ölartige Rest, die Ester-Base der Formel V wird in 170 ml Azeton aufgelöst, bis zum Sieder erhitzt, wonach zur erhaltenen Lösung 4,82 g Oxalsäure, d.h. eine Lösung der letzteren in 170 ml Azeton zugegeben wird. Das Gemisch wird während 30 Minuten unter Umrühren zum Sieden erhitzt, wonach es bei Zimmertemperatur zum Auskristallisieren während 16 bis 17 Stunden stehengelassen wird.

Die gebildeten Kristalle des Oxalatsalzes des Diesters IV werden abfiltriert, mit Azeton gespült und danach 50 bis 60°C getrocknet. Es wird 19,3 g technisches Oxalat erhalten, welches zweimal aus einem Azeton-Wasser-Gemisch (9 zu 1) umkristallisiert. Ausbeute des umkristalisierten Oxalats -8 g mit einem Schmelzpunkt von 117. bis 120°C. Lit. Schmelzpunkt 117-121°C>

$^1$H-kernmagnetische Resonanz Spektrum der Diester-Base mit Formel IV / $CD_3CN$, Spektrometer mit einer Frequenz von 100 MHz/δ ; ppm 1,10-1,56 (m,6H) : $(CH_2)_3$ ; 2,14-3,18 (m.20 H) : 2x $NCH_2CH_2CO$ , 2x$ArCH_2$ , 2x 3-C 2x 4-$CH_2$ ; 3,66-4,04 (m,6H) :2x 1-CH und 2x $COOCH_2$ ; 3,48 - (s,6H) : 2x $CH_3O$ ; 3,65 (s,18H) : 6x $CH_3O$ ; 6,16 - (s,2H) : 2x 8-CH ; 6,46 (s,2H) 2x 5-CH ; 6,58-6,76 - (m,6H) : 6xArH.

Die Daten vom Spektrum stimmen mit diesen der Literatur (1) überein. $^1$H-kernmagn.Resonanz Spektrum des Diesteroxalat (DMSO-$\alpha_6$) : 1,12-1,64 m,6H) : $(CH_2)_3$ ; 2,58-3,66 (m,20 H) : 2x $NCH_2CH_2CO$, 2x $ArCH_2$ : 2x3 -$CH_2$, 2x 4-$CH_2$; 3,88 - (m,4H) : 2x$COOCH_2$ ; 4,25 (m,2H) : 2x1-CH ; 3,36 - (s, 6H) 2x$CH_3O$ ; 3,62 (s,6H) : 2x $CH_3O$ ; 3,65 (s,12H) : 4x $CH_3O$ ;5,90 (s,2H) : 2x 8-CH ; 6,64 (s, 2H) : 2x 5-CH ; 6,42-6,92 (m, 6H) : 6x ArH ; 6,15 - (erweitertes Singulett 4H, wird mit $D_2O$ ausgetauscht) : 2x (COOH)

Beispiel 3. Herstellung von N,N-Dimethyl-N,N-4,10-dioxa-3,11-dioxotridezylen-1,13-tetrachydropapaverinoil-dichlorid.

Es werden 0,5 g (0,00042 Mol) Dijodid der Formel V in 20 ml trockenem Azetonitril aufgelöst und die erhaltene Lösung wird durch eine mit 2 g - (4,4, mg Äqv.) Amberlist A-27 ($Cl^{\ominus}$)® gefüllte Säule mit einer Geschwindigkeit von 10 ml/Stunde durchgelassen. Die Säule wird mit 40 ml trockenem Azetonitril eluiert. Das erhaltene Eluent wird unter Vakuum bei einer Temperatur von 25 bis 30°C bis zu einem Volumen von 5 ml konzentriert und unter Umrühren tropfenweise zu 200 ml trockenem Äther zugegeben. Es wird bei 50 bis 60°C getrocknet. Ausbeute der I : 0,398 g (94%) mit einem Schmelzpunkt von 122-128°C.

Elementenanalyse : berechnet in % für $C_{53}H_{72}Cl_2O_{12}$ . 4,5 $H_2O$

N -2,59

gefunden: N -2,48

$^1$H-kernmagnetische Resonanz ($CD_3CN$) : 1,28-1,70 (m, 6H) : $(CH_2)_3$ ; 2,98 (s) und 3,23 (s) : 2x N-$CH_3$ (von Isomeren A und B) ; 2,76-3,96 m, 20H) : 2x $NCH_2CH_2CO$ , 2x $ArCH_2$ , 2x 3-$CH_2$ , 2x 4-$CH_2$ ; 3,60 (s 6H) : 2x $CH_3O$ ; 3,66 (s,18H) : 6x $CH_3O$ ; 3,96-4,12 (m,4H): 2x $COOCH_2$ 4,68 (m) und 4,88 - (m) : 2x 1-CH (von Isomeren A und B, Verhältnis 29,5 : 70,5) ; 5,61 (s) und 5,78 (s) : 2x 8-Ch (von Isomeren A und B, Verhältnis 29,5 : 70,5) ; 6,30-6,76 (m, 8H) : 2x 5-CH und 6x ArH.

Bestimmen der Reinheit der Substanz durch die hocheffektive Flüssigchromatografie:

Chromatografische Bedingungen : Säule mit umgekehrter Phase $C_{18}$, bewegliche Phase wässrige 0,1 M Kaliumhydrogenphosphat, enthaltend Natriumoktylhydrogensulfat (ein g/l) und Azetonitril in einem Verhältnis 1:1. Durchlaßgeschwindigkeit 1,5 1/Min.

Gehalt an aktiver Substanz: 98% ; Gehalt an Chlormethylat des N-(2-Carboxyäthyl)-1,2,3,4-tetrahydropapaverin : 1,95% Gehalt an Laudinozin : Spuren unter 0,1%.

Vergleichsreinheit des nach demselben Schema synthesierten Bezylats : Gehalt an aktiver Substanz -92% Nebenprodukte von: N-Methyl-N,N-4,10-dioxa-3,11-dioxotridezylen-12-en-1-tetrahydropapaverin-benzolsulfonat, N-Methyl-N-(2-carboxyäthyl)-1,2,3,4-tetrahydropapaverin-benzolsulfonat, Laudanozin, N-(9-Hydroxy-4-oxanoil-3-oxo)-N-methyl-1,2,3,4-tetrahydropapaverin-benzolsulfonat, N,N-Dimethyl-1,2,3,4-tetrahydropapaverin-benzolsulfonat und N-Methyl-N,N-4,10-dioxa-3,11-dioxotridezylen-1,13-bis-tetrahydropapaverin-benzolsulfonat - insgesammt 8%.

Diese Daten stimmen mit den Literaturdaten über die Reinheit und Gehalt an Nebenprodukten in dem bekannten Bezylat überein (1,2,3).

Beispiel 4. Vergleichsprüfung der Stabilität des Dichlorids I und des Bezylats mittels der hocheffektiven Flüssig-Chromatografie.

Chromatografische Bedingungen: Säule mit umgekehrter Phase $C_{18}$, bewegliche Phase: wässrige 0,1M Kaliumhydrogenphosphat, enthaltend Natriumoktylhydrogensulfat (1 g/l) und Azetonitril in einem Verhältnis 1:1. Durchlaßgeschwindigkeit 1,5 ml/Min.

Es werden je 7 Proben einer 0,5%-igen wässrigen Lösung des Dichlorids I und des Dibezylats mit bestimmten pH-Werten zubereitet, was durch Zufügen von Ortophospharsäure ereicht wird. Die Proben verweilen 40 Stunden bei einer Temperatur von 53°C. Danach wird der Prozentgehalt an Dichlorid und Dibezylat in den Proben gemessen. Die Ergebnisse sind in Tabelle 1 veranschaulicht.

Beispiel 5. Pharmakologische Prüfungen

Die Versuche wurden an Katzen unter Einführen von gleichen Dosen des Originalpräparats "Tracrium" und der Verbindung der Formel I. Es wurde das klassische Verfahren"Ischiadiensgastrocnemicus" benutzt. Es wurde eine Kontraktion des Muskels Gastrocnemicus zufolge einer Reizung des Hüftnervs (Ischiasnervs) registriert. Die Ergebnisse sind in Tabelle II dargestellt.

Die durchgeführten Versuche zeigen, daß die Resultate des Eintretens, der Wiederherstellung und der Dauer des Effekts der Neuromuskelübertragung unter der Wirkung des neusynthetisierten Dichlorids der Formel I und des Orgiginalpräparates "Tracrium" gleich sind und keinen statistisch wesentlichen Unterschied in deren Wirkung beobachtet wird.

## PHARMAZEUTISCHE ZUSAMMENSETZUNG

Beispiel 6. Herstellung einer Ampullenform zur intravenösen Anwendung:

| | |
|---|---|
| Dichlorid der Formel I | 1 g |
| Bakteriostatisches Agens | 0,01 g |
| Natriumdihydrogenphosphat | 0,02 g |
| Verdünnte Phosphorsäure bis zum entsprechenden pH-Wert (3,0-3,5) | |
| Wasser für Injektionen bis | 100 ml |

Die beispielsweise Zusammensetzung wird nach dem aseptischen Verfahren hergestellt. In zirka 80 ml sterilem Wasser für Injektionen werden nacheinander das bakteriostatische Agens, das Natriumdihydrogenphosphat und das Dichlorid I aufgelöst. Die Lösung wird bis zu einem pH-Wert 3,4 mit verdünnter Phosphorsäure korrigiert und bis zu einem bestimmten Volumen abgeglichen. . Die fertige Lösung wird durch eine Membranfiltrierung durch einen Filter mit einer Porengröße von 0,22 µ.sterilisiert. Die filtrierte Lösung wird in Ampullen von 2 ml oder 5 ml gefüllt, wonach die Ampullen aseptisch zugelötet werden.

Beispiel 7 Herstellung einer Lyophilen Form.

| | |
|---|---|
| Dichlorid mit Formel I | 1,00 g |
| Bakteriostatisches Agens | 0,01 g |
| Mannitol | 2,00 g |
| Natriumdihydrogenphosphat | 0,02 g |
| Verdünnte Phosphorsäure bis zu einem pH-Wert | 3,0-3,5 |
| Wasser für Injektionen bis | 100 ml |

Die Injektionslösung wird aseptisch nach dem obenbeschriebenen Verfahren hergestellt, wobei nach dem Auflösen des bakteriostatischen Agens auch Mannitol beigefügt wird. Die gefilterte Lösung wird in Ampullen oder Flakons von 2 oder 5 ml gefüllt, welche lyophilisiert werden.

TABELLE 1

PRÜFUNG DER STABILITÄT DES DIBENZYLATS UND DES DICHLORMETHYLATS DER FORMEL I IN WÄSSRIG-SAUREM MEDIUM BEI 53°C WÄHREND 40 STUNDEN

| | pH-Werte | | | | | | | | | | |
|---|---|---|---|---|---|---|---|---|---|---|---|
| | 1,76 | 2,46 | 2,75 | 2,88 | 3,04 | 3,16 | 3,27 | 3,50 | 3,58 | 3,71 | 4,09 |
| Dibezylat Prozent-gehalt | 60,65 | | | 72,86 | | 73,45 | | 73,79 | | 72,16 | 68,8 |
| Dichlor-methyl der Formel I Prozent-gehalt | | 88,1 | 89,5 | | 91,9 | | 91,4 | | 87,6 | | 70,4 |

TABELLE 2

VERGLEICHSTABELLE FÜR DAS EINTRETEN UND DIE WIEDERHERSTELLUNG DER NEUROMUSKEL-ÜBERTRAGUNG UNTER DER WIRKUNG DES DICHLOR-METHYLAT DER FORMEL I UND "TRACRIUM", EINGEFÜHRT IN GLEICHEN DOSEN

| Präparate Dosis mg/kg $E_{95}$ | Eintreten der Neuro-muskel-Blockierung (Sek.) | Wiederherstellung der Neuromuskel-Übertragung (Min.) |
|---|---|---|
| Dichlormethylat der Formel I 0,2 | 60,3 | 29,8 |
| "Tracrium" 0,2 | 60,4 | 34,6 |

**Ansprüche**

1. Dichlormethylat des N,N-4,10-Dioxa-3,11-dioxotridezylen-1,13-bis-tetrahydropapaverin der allgemeinen Formel I

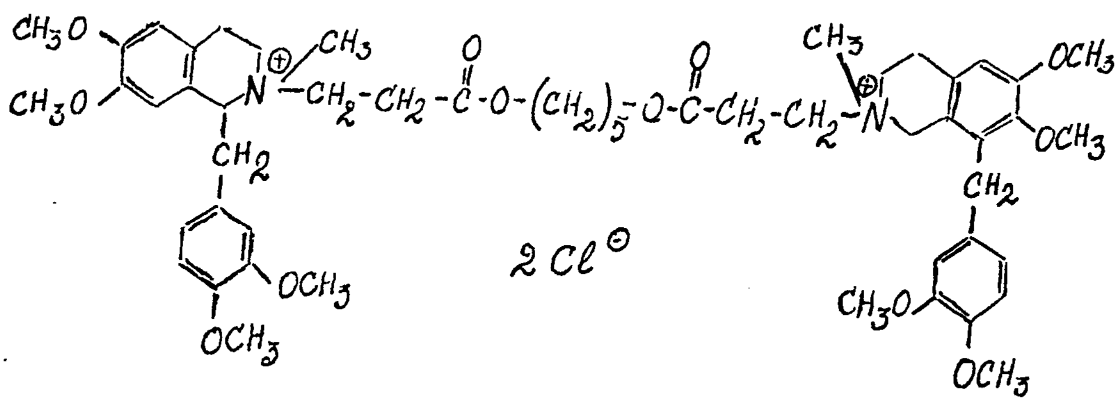

2. Verfahren zur Herstellung einer Verbindung gemäß Patentanspruch 1 dadurch **gekennzeichnet**, daß 1,5-Pentamethylendiol mit N-(2-Carboxyäthyl)-1-(3,4-dimethoxybenzyl)-6,7-dimethoxy-1,2,3,4-tetrahydroisochinolin der allgemeinen Formel II verestert wird

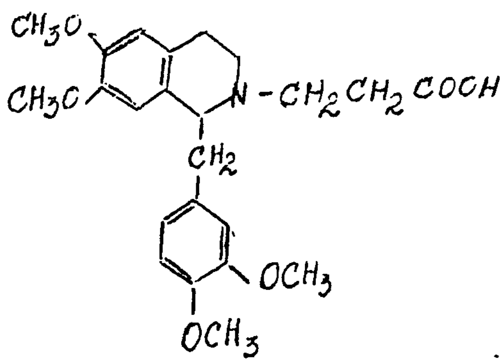

II

in Anwesenheit von sauren Katalysatoren in einem Medium aromatischer Kohlenwasserstoffe unter azeotroper Abscheidung des Reaktionswassers bis zum Erhalten des Diesters, welcher mit Methyljodid bis zu N,N-Dimethyl-N,N-4,10-dioxa-3,11-dioxotridezylen-1,13-bis-tetrahydropapaverinoil-dijodid quaternärisiert wird und das letztere in eine Verbindung der Formel I mittels Anionenaustausch unter Verwendung von starkbasischen Anionenaustauscher-Harzen umgesetzt wird.

3. Verfahren gemäß Patentanspruch 2, dadurch **gekennzeichnet**, daß die sauren Katalysatoren organische, anorganische oder Lewis-Säuren sind, vorzugsweise p-Toluolsulfonsäure.

4. Verfahren gemäß Patentanspruch 2, dadurch **gekennzeichnet** , daß die Veresterung des 1,5-Pentamethylendiols mit einem Überschuß der Verbindung der Formel II in Molverhältnissen Säure zu Diol von 2:1 bis 4:1 durchgeführt wird.

5. Verfahren gemäß Patentanspruch 3, dadurch **gekennzeichnet**, daß das Molverhältnis der p-Toluolsulfonsäure zu Verbindung der Formel II 1:1 bis 2:1 beträgt.

6. Verfahren gemäß Patentanspruch 2, dadurch **gekennzeichnet**, daß die starkbasischen Anionen-Austausch-Harze Dowex ($Cl^{\ominus}$)®, Amberlits ($Cl^{\ominus}$)®, Amberlists ($Cl^{\ominus}$)®, vorzugsweise Amberlist A-26 -($Cl^{\ominus}$)®, Amberlist A-27 ($Cl^{\ominus}$)® sind.

7. Verfahren gemäß Patentanspruch 2, dadurch **gekennzeichnet**, daß als Eluente beimAnionenaustausch aliphatische Alkohole, aliphatische Ketone oder aliphatische Nitrile verwendet werden.

In Betracht gezogene Druckschriften

1. GB-Patentschrift No 1 579 822

2. Stenlake J.,Waigh R., Dewar G., Eur.J.Med.chem. 1981, 16(8), 515-524

3. Chapple D.Y., Clark J.S., Br. J. Anaesth., 1983, 55, 119

4. Carthy B.J., Hill G.T., Analytical Proceeding, 1983, 177

5. Stenlake J.B., Waigh R.D.,Dewar G.H., Corer G.G. Br.J.Anaesth, 1983, 39,55

Europäisches Patentamt

# EUROPÄISCHER RECHERCHENBERICHT

Nummer der Anmeldung

EP 86110018.8

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| Y,D | GB - A - 1 579 822 (THE WELLCOME FOUND.)<br>* Ansprüche 1,8,16,26; Seite 2, Zeile 51 - Seite 3, Zeile 5; Beispiel 1 *<br>-- | 1,2 | C 07 D 217/20<br>// A 61 K 31/485 |
| Y | DE - A1 - 3 039 304 (THE WELLCOME FOUND.)<br>* Ansprüche 1,8; Verfahren 1, Seite 20; Seite 29, Zeilen 18-20 *<br>-- | 1,2 | |
| A | EP - A1 - 0 010 119 (MASSACHUSETTS G. HOSP.)<br>---- | | |

RECHERCHIERTE SACHGEBIETE (Int. Cl.4)

C 07 D 217/00

Der vorliegende Recherchenbericht wurde für alle Patentansprüche erstellt.

| Recherchenort | Abschlußdatum der Recherche | Prüfer |
|---|---|---|
| WIEN | 23-10-1986 | HOCHHAUSER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument